# EUROPEAN PATENT APPLICATION

(11) **EP 1 358 866 A2**
(43) Date of publication of application: **05.11.2003**
(21) Application number: 03252695.6
(22) Date of filing: 29.04.2003
(51) Int. Cl.: A61K 7/06, A61K 7/02, A61K 7/48

(54) **Cosmetic compositions comprising at least one dimethicone, at least one linear hydrocarbon wax and at least one compatibilizing agent**

(30) Priority: 29.04.2002 US 375814 P
(71) Applicant: L'OREAL S.A., 75008 Paris Cedex (FR)
(72) Inventor: Yu, Wei, Edison, New Jersey 08820 (US)
(74) Representative: Bentham, Stephen

(57) **Abstract**

Compositions and methods for making and using compositions comprising an emulsion comprising at least one dimethicone, at least one linear hydrocarbon wax, and at least one compatibilizing agent. In one embodiment, the emulsions are capable of being molded into sticks.

## Description

In an embodiment, the present invention relates to a composition comprising an emulsion comprising at least one dimethicone, at least one linear hydrocarbon wax, and at least one compatibilizing agent.

Cosmetic and/or dermatological products may comprise a structured, *i.e*., gelled and/or rigidified, liquid fatty phase, such as in solid compositions, for example, deodorants, lip balms, lipsticks, concealer products, eyeshadows, and cast foundations. This structuring may be obtained with the aid of waxes and/or fillers. Unfortunately, waxes and fillers may have a tendency to make the composition matte and/or uncomfortable (*e.g*., tight and/or dry) after application to keratinous material, which may not always be desirable, in particular for a lipstick or an eyeshadow.

Furthermore, it is desirable that care, treatment, and make-up compositions have good staying power or long wearing properties over time. Poor staying power is characterized by a color change (turning, fading) or a nonuniform change in the make-up effect over time, generally following an interaction with sebum and/or sweat secreted by the skin, and, for the lips, an interaction with saliva. Specifically, a composition which does not have good staying power or long-wearing properties over time may oblige the user to reapply the make-up regularly. However, consumers nowadays often wish to enhance the beauty of their face or body while spending as little time as possible in doing so.

Accordingly, consumers may be interested in a product in stick form which has at least one of the following properties: depositing a film with good staying power and/or long wearing properties, glossiness, and wear comfort, for example, non-desiccating and not tightening.

The aforementioned properties are generally associated with the nature of the liquid fatty phase. Thus, attempts have been made to reduce the amount of waxes and/or fillers in the composition to increase the gloss. Often, however, that may lead to an increase in the migration of the liquid fatty phase, *i.e.,* a film with poor staying power and/or poor long wearing properties. Further, the glossiness and/or wear comfort of the product may be reduced. In other words, the amounts of waxes and of fillers required to prepare a stick of suitable hardness which does not exude at room temperature are often a restricting factor on at least one desirable property of the deposit.

Attempts have also been made to formulate products which can be applied in combination, *i.e.,* before, after, and/or simultaneously, with at least one composition, for example, possessing at least one of the aforementioned undesired characteristics. For example, cosmetic compositions have been formulated to act as topcoat compositions, *i.e*., compositions which are applied on top of another cosmetic composition usually referred to as a "basecoat." The goal of such topcoat compositions has been to improve certain properties of the basecoat. For example, U.S. Patent No. 6,019,962 discloses emulsions and methods for using the emulsions to improve the performance of long wearing cosmetic products and to enable the user to significantly enhance the attributes of long-wearing cosmetic products without compromising their primary advantages, such as gloss and feel. Commonly, however, topcoat products are liquids and may not be as convenient to use and/or may not have at least one desired characteristic, such as non-stickiness and non-migratory properties, which may be provided to a user with a stick composition.

Accordingly, there is a need for a cosmetic composition in stick form which may, for example, deposit a film on at least one keratinous material, the film having at least one property chosen from good staying power, long wearing properties, glossiness, stability, and wear comfort.

The present inventors have surprisingly discovered that a composition comprising an emulsion comprising at least one dimethicone, at least one linear hydrocarbon wax, and at least one compatibilizing agent may in some embodiments form a stick which deposits a film having at least one of the aforementioned desirable properties. As used herein, a composition comprising an emulsion includes a composition comprising more than one emulsion.

Thus, in an embodiment, the present invention provides an emulsion comprising at least one dimethicone, at least one linear hydrocarbon wax, and at least one compatibilizing agent. In one embodiment, the emulsion is capable of being molded in the form of a stick.

In one embodiment, the emulsions of the present invention comprise two incompatible phases, at least one dimethicone and at least one linear hydrocarbon wax, that are stabilized in an emulsion by the at least one compatibilizing agent.

In an embodiment, the present invention provides a method for modifying the structure of a composition comprising at least one dimethicone and at least one linear hydrocarbon wax, comprising the addition of at least one compatibilizing agent in an amount effective to mold the composition into a stick.

In an embodiment, the inventive composition and/or emulsion is non-aqueous. In an embodiment, the inventive composition is capable of being molded in the form of a stick and the stick is stable. In an embodiment, the inventive composition further comprises at least one low viscosity dimethicone different from the at least one dimethicone. In an embodiment, the inventive emulsion is a lip product. In an embodiment, the lip product is a lipstick. In another embodiment, the lip product is a topcoat.

Certain terms used herein are defined below:

"At least one" means one or more and thus includes individual components as well as mixtures/combinations.

"Non-aqueous," as used herein, refers to compositions comprising less than 5% water by weight relative to the total weight of the composition, such as less than 2% water by weight, and further such as less than 1% water by weight. The amount of water in the composition for purposes of this invention refers to the amount of water added to the components of the composition.

"Emulsion," as used herein, refers to a system of two or more incompatible materials, in which one material is suspended or dispersed throughout another material in separate droplets and is stable.

"Incompatible" compounds, as used herein, refers to compounds which, when combined in the absence of the at least one compatibilizing agent, result in an emulsion which is not homogeneous as viewed by the naked eye. For example, compounds are incompatible when, after an emulsion comprising the compounds is heated with mixing to a temperature which is equal to the melting point of highest melting compound in the emulsion and the temperature of the emulsion is then maintained for 5 minutes without mixing, the emulsion is cloudy to the naked eye and/or comprises at least two separate phases as viewed by the naked eye.

"Gloss," as used herein, refers to surface shininess. The gloss of a composition is measured and evaluated using a gloss meter. Gloss meters are commonly used in the nail polish art, and measure the amount of light reflected from the surface or film of interest. The gloss may be quantified, for example, as a % reflectance.

"Staying power," as used herein, includes long wear properties and refers to the ability of a film of a composition deposited on a keratinous material to retain at least one of: color (including shade and intensity), gloss, and thickness. These properties are evaluated and compared visually, such as by applying a film of a composition to at least one human keratinous material and inspecting these properties after a predetermined amount of time as described in the example.

"Wear comfort," as used herein, refers to at least one property exhibited by a film of a composition deposited on at least one keratinous material chosen from non-desiccating, not tightening non-drying, moisturizing, non-greasy and non-stickiness. These properties are evaluated and compared visually, such as by applying a film of a composition to at least one human keratinous material and inspecting these properties after a predetermined amount of time as described in the example.

"Hydrocarbon group," as used herein, includes substituted linear alkyl groups, unsubstituted linear alkyl groups, substituted branched alkyl groups, unsubstituted branched alkyl groups, substituted cyclic alkyl groups, unsubstituted cyclic alkyl groups, substituted linear alkenyl groups, unsubstituted linear alkenyl groups, substituted branched alkenyl groups, unsubstituted branched alkenyl groups, substituted cyclic alkenyl groups, unsubstituted cyclic alkenyl groups, substituted aromatic groups, and unsubstituted aromatic groups, wherein the aforementioned unsubstituted groups comprise only carbon and hydrogen atoms.

"Substituted," as used herein, means further comprising at least one substituent. Non-limiting examples of substituents include atoms, such as oxygen, nitrogen, and halogens, as well as functional groups, such as hydroxyl, ether groups, oxyalkylene groups, polyoxyalkylene groups, carboxylic acid groups, amine groups, and amide groups.

"Migration," as used herein, means a running of the composition beyond the initial application line as viewed by the naked eye.

"Keratinous fibers," as used herein, includes hair (including eyelashes and eyebrows).

"Keratinous material," as used herein, includes skin (including lips), hair (including eyelashes and eyebrows), and nails.

"Polymer," as used herein, comprises copolymers and homopolymers, including but not limited to, for example, block polymers, cross linked polymers, and graft polymers.

"Copolymer," as used herein, refers to polymers formed from at least two different types of monomers.

"Silicone polymers," as used herein, includes, for example, siloxanes, organosilanes and organosiloxanes; and refers to a polymer comprising at least one monomeric unit comprising at least one silicon atom and at least one oxygen atom, wherein the polymer may be chosen from linear polymers, branched polymers, and cyclic polymers; further wherein the polymer may optionally be substituted with at least one group different than said at least one hydrocarbon group; further wherein the polymer may optionally further comprise at least one heteroatom different from silicone atoms and oxygen atoms intercalated in the polymer chain.

A composition that is "capable of being molded," as used herein, refers to compositions which are pourable or shapeable when heated but which set or harden as cooled. For example, a composition capable of being molded may be pourable or shapeable when it is heated but, once cooled to room temperature, the composition retains its shape. Whether a composition is "capable of being molded," as defined herein, is tested by determining whether a composition, upon heating to 37°C or greater (such as, for example to a temperature ranging from 50°C to 75°C) is pourable or shapeable and whether, upon subsequent cooling, it retains its shape.

The term "stable" refers to a composition which, under prescribed conditions, does not exhibit at least one abnormality in the composition such as, for example, bending or leaning if the composition is in stick form, phase separation, melting, or syneresis.

Stability is tested by placing the composition in a controlled environment chamber for a specific amount of time at a specific temperature. Compositions in the form of a stick are tested standing up in the chamber. "Standing up," as used herein; means upright, in a vertical position. For example, if the composition is in the form of a stick, the stick is placed upright in the chamber, *i.e*., in a vertical position with respect to the surface of the chamber on which the stick is placed.

According to the present invention, a composition is stable if it does not exhibit at least one abnormality in the emulsion after 2 months at room temperature (25°C.) as viewed by the naked eye. However, more stringent requirements for stability may be used depending on the intended use of the composition. For example, in an embodiment, a composition is stable if it does not exhibit at least one abnormality in the emulsion after 6 months at room temperature (25°C) as viewed by the naked eye. In another embodiment, a composition is stable if it does not exhibit at least one abnormality in the composition after 9 months at room temperature (25°C) as viewed by the naked eye. In another embodiment, a composition is stable if it does not exhibit at least one abnormality in the composition after 4 weeks at 37°C as viewed by the naked eye. In another embodiment, a composition is stable if it does not exhibit at least one abnormality in the composition after 4 weeks at 50°C as viewed by the naked eye.

In these stability tests, the physical condition of the sample is inspected as it is placed in the chamber. The sample is then inspected after a specific length of time after the sample is placed in the chamber, such as 8 hours, 12 hours, 24 hours, 1 week, 2 weeks, 3 weeks, 4 weeks, 5 weeks, 6 weeks, 7 weeks, 8 weeks (2 months), 12 weeks (3 months), 16 weeks (4 months), 20 weeks (5 months), 24 weeks (6 months), 28 weeks (7 months), 32 weeks (8 months), 36 weeks (9 months), and/or 56 weeks (1 year). The temperature of the chamber is set at a specific temperature, such as 25°C, 37°C, or 50°C, as described above, or, as further examples, at 4°C for 1 year, or at 45°C for 4 weeks. For example, as discussed above, according to the present invention, a composition is stable if it does not exhibit at least one abnormality in the emulsion after 2 months at room temperature (25°C.) as viewed by the naked eye. Thus, the physical condition of the composition is inspected as it is placed in a chamber set at 25°C, inspected after specific lengths of time, if desired, and ultimately, inspected after 2 months.

In another embodiment, a sample may be tested for at least one abnormality under freeze-thaw conditions, where the sample is frozen for 12 hours and then allowed to thaw for 12 hours. Generally, freeze-thaw conditions comprise 3 cycles of each of the aforementioned 12 hour periods.

At each inspection, the sample is examined for at least one abnormality in the emulsion such as, for example, bending or leaning if the emulsion is in stick form, phase separation, melting, or syneresis. As used herein, "syneresis" is the appearance of droplets on a surface of an emulsion that are visible to the naked eye.

The skilled artisan will readily recognize at least one abnormality that impedes functioning of a composition based on the intended application, such as, for example, the appearance of at least one abnormality as described above. The skilled artisan will also readily recognize that the observation of at least one abnormality that impedes functioning of a composition will depend not only on its intended application, but its composition as well.

It is to be understood that both the foregoing general description and the following detailed description are exemplary and explanatory only and are not restrictive of the invention as claimed. Reference will now be made in detail to exemplary embodiments of the present invention.

As previously discussed, the present invention, in an embodiment, provides a composition comprising an emulsion comprising at least one dimethicone, at least one linear hydrocarbon wax, and at least one compatibilizing agent. In an embodiment, the inventive emulsion is chosen from aqueous emulsions and non-aqueous emulsions. In an embodiment, the inventive composition is a cosmetic composition. In an embodiment, the inventive composition further comprises at least one low viscosity dimethicone different from the at least one dimethicone.

The moldable compositions of the present invention may, for example, make it possible to form a product having a desired shape, such as for example a stick form or the form of a container, such as a compact form. Further, for example, the moldable compositions may make it possible to control the exudation of components that form the compositions, and thus may aid in the storage of such compositions and in the application of cosmetic, dermatological and therapeutic compositions on at least one keratinous material.

Accordingly, the present invention applies not only to make-up products for at least one keratinous material such as lip compositions, lip pencils, foundations including foundations which may be cast in the form of a stick, a dish, a pan or a jar, concealer products, temporary tattoo products, eyeliners, mascara bars, but also to body hygiene products such as deodorant sticks, and to care products and products for treating at least one keratinous material, such as sunscreen and anti-sun products which may be in stick form. Thus, the present invention may be in the form of mascara product including mascara bars, an eyeliner product, a foundation product, a lipstick product, a blush for cheeks or eyelids, a deodorant product, a make-up product for the body and/or hair, a make-up-removing product, an eyeshadow product, a face powder product, a concealer product, a treating shampoo product, a hair conditioning product, a sunscreen, colorant for the skin or hair, or skin care formula such as, for example, anti-pimple or shaving cut formulas.

In one embodiment, the at least one compatibilizing agent of the present invention may make it possible to obtain an emulsion of at least one dimethicone and at least one linear hydrocarbon wax such as to enable formation of a stable stick product. Also, in addition to making it possible to obtain a product in the form of a stick or tube, the inventive emulsion may limit the exudation of a fatty phase from the solid emulsions, especially in hot and humid regions; may limit, after deposition on keratinous material, such as skin or lips, the migration of this phase into wrinkles and fine lines; and/or may deposit a film on keratinous material which has at least one property chosen from good staying power, long wearing properties, glossiness, and wear comfort.

These characteristics are particularly sought after in cosmetic compositions, such as lip products, such as lipsticks, concealer products, and eyeshadows. Specifically, significant migration of the liquid fatty phase, in particular when it is charged with coloring agents, may lead to an unpleasant appearance around the lips and the eyes, *i.e*., making the wrinkles and fine lines appear more prominent. This migration is often mentioned by consumers as being a major defect of conventional lip products, concealer products and eye make-ups.

Accordingly, in an embodiment, the present invention is drawn to a care and/or make-up and/or treatment compositions for the skin and/or the lips of the face and/or for superficial body growths such as keratinous fibers which may make it possible to overcome at least one of the drawbacks mentioned above.

The inventive compositions may be modified according to the nature of the at least one compatibilizing agent, the viscosity of the at least one dimethicone, and the nature of the at least one linear hydrocarbon wax, and may be such that a rigid structure in the form of a tube or stick with mechanical strength is obtained. When these tubes or sticks are colored, they may make it possible, after application, to obtain a uniformly, *i.e*., homogeneously, colored glossy deposit, such as a layer, which does not migrate, for example, into the wrinkles and fine lines of the skin surrounding, for example, the lips and eyes, and which has good staying power and/or long-wearing properties, in particular of the color, over time. Thus, the compositions of the invention may, for example, be a composition for the skin or the lips, such as a foundation composition, concealer product, eyeshadow or lipstick composition, *e.g*., in stick form.

In another embodiment, the present invention provides a non-aqueous composition comprising an emulsion comprising at least one dimethicone, at least one linear hydrocarbon wax, and at least one compatibilizing agent. In another embodiment, the present invention is drawn to a method for making a stable emulsion comprising combining at least one dimethicone, at least one linear hydrocarbon wax, and at least one compatibilizing agent, wherein the at least one compatibilizing agent is present in an amount effective to provide an emulsion which is capable of being molded in the form of a stable stick.

In another embodiment, the present invention provides a method for caring for, making up and/or treating at least one keratinous material comprising applying to the at least one keratinous material a composition comprising an emulsion comprising at least one dimethicone, at least one linear hydrocarbon wax, and at least one compatibilizing agent. In another embodiment, the composition further comprises at least one low viscosity dimethicone different from the at least one dimethicone.

The emulsion of the invention can be a single or multiple emulsion, such as an oil-in-water or water-in-oil emulsion or an oil-in-water-in-oil emulsion, or a water-in-oil-in-water emulsion, or a rigid or soft gel containing an oily continuous phase. In an embodiment, the inventive emulsion is in a form cast as a stick or in a dish, for example, in the form of an oily rigid gel, such as an anhydrous gel, e.g., an anhydrous stick. In a further embodiment, the emulsion is in the form of an opaque or translucent rigid gel (depending on the presence or absence of pigments). In another embodiment, the emulsion of the invention is self-supporting. In another embodiment, the emulsion of the invention is a non-aqueous emulsion.

In an embodiment, the present invention is drawn to a method of providing at least one property chosen from staying power, long wear properties, glossiness, and wear comfort comprising applying to at least one keratinous material a composition comprising an emulsion comprising at least one dimethicone, at least one linear hydrocarbon wax, and at least one compatibilizing agent.

In an embodiment, the inventive composition is a topcoat product. For example, the present invention may be a method of increasing at least one property of a basecoat composition chosen from properties of staying power, long wear properties, glossiness, and wear comfort comprising applying to at least one keratinous material at least one basecoat composition; and applying to the basecoated keratinous material a composition of the invention.

Non-limiting examples of dimethicone according to the present invention include Dow Corning 200 (350 cst) or dimethicones of viscosity equal to or higher than 100 cst, such as, for example, 100, 350, and 500 cst. All viscosities used herein are measured using the same parameters which obtain 350 cst for Dow Corning 200.

In an embodiment, dimethicone is present in the composition in an amount ranging from 5% to 95% by weight relative to the total weight of the composition. In another embodiment, dimethicone is present in an amount ranging from 10% to 90% by weight relative to the total weight of the composition. In another embodiment, dimethicone is present in an amount ranging from 20% to 80% by weight relative to the total weight of the composition.

The at least one linear hydrocarbon wax according to the present invention is solid at room temperature, and may have a melting point greater than about 35°C, such as, for example greater than about 55°C. In an embodiment, the at least one linear hydrocarbon wax is incompatible with high viscosity dimethicone, such as, for example, 100, 350, and 500 cst. Non-limiting examples of at least one linear hydrocarbon wax include polyethylene waxes and -linear paraffin waxes. According to the present invention, the linear hydrocarbon of the at least one linear hydrocarbon wax may be chosen from substituted linear alkanes, unsubstituted linear alkanes, substituted linear alkenes, and unsubstituted linear alkenes, wherein the aforementioned unsubstituted groups comprise only carbon and hydrogen atoms.

Non-limiting examples of the at least one linear hydrocarbon wax include polyethylene waxes such as Polyethylene 400 and Polyethylene 500, which are commercially available from New Phase Technologies, linear paraffin waxes such as paraffin S&P 206, S&P 173, and S&P 434, which are available from Strahl & Pitsch, and long-chain linear alcohols such as products comprising blends of long-chain linear alcohols and polyethylene such as Performacol 425 (C₂₀-C₄₀ alcohols and polyethylene) and Performacol 550 (C₃₀-C₅₀ alcohols and polyethylene), which are commercially available from New Phase Technologies.

In an embodiment, the at least one linear hydrocarbon wax is present in the composition in an amount ranging from 2% to 30% by weight relative to the total weight of the emulsion. In another embodiment, the at least one linear hydrocarbon wax is present in an amount ranging from 5% to 20% by weight relative to the total weight of the composition.

The at least one compatibilizing agent according to the present invention is chosen from silicone polymers comprising at least one hydrocarbon group. In an embodiment, at least one of the at least one hydrocarbon groups comprises at least 18 carbon atoms.

In an embodiment, the at least one compatibilizing agent is chosen from compatibilizing agents of formula (I). Compatibilizing agents of formula (I) have the following structure: 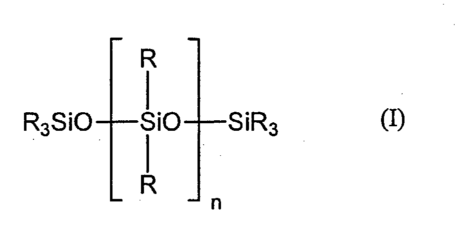 wherein
R, which may be identical or different, are each chosen from hydrocarbon groups; and
the total molecular weight of Si and O atoms is at least 10% of the total weight of the at least one compatibilizing agent of formula (I), and
the melting point of the at least one compatibilizing agent of formula (I) ranges from 35°C to 110°C
with the proviso that at least one R comprises at least 18 carbon atoms.

In an embodiment, at least one R is chosen from C₃₀ to C₄₅ hydrocarbon groups. In another embodiment, at least one R is chosen from C₃₀ to C₄₅ alkyl groups. For example, the at least one compatibilizing agent may be chosen from at least one C₃₀-C₄₅ alkyl dimethicone. In another embodiment, the at least one compatibilizing agent is solid at room temperature.

Non-limiting examples of suitable silicone polymers comprising at least one hydrocarbon group include linear polysiloxanes, branched polysiloxanes, cyclic linear polysiloxanes. Non-limiting examples of the at least one compatibilizing agent include linear polysiloxanes comprising at least one C₃₀-C₄₅ alkyl group, such as SF1642 sold by GE Silicones; C₃₀-C₄₅ alkyl methicones, such as AMS-C30 wax sold by Dow Corning; C₂₀-C₂₄ alkyl methicones, such as SilCare 41M40 sold by Clariant; C₂₄-C₂₈ alkyl methicones, such as Abil Wax 9810 sold by Goldschmidt and SilCare 41 M50 sold by Clariant; hexyl methicones, such as SilCare 41M10 sold by Clariant; caprylyl methicones, such as SilCare 41M15 sold by Clariant; lauryl methicones, such as SilCare 41M20 sold by Clariant; stearyl methicones, such as SilCare 41M30 sold by Clariant; cetyl dimethicone sold by Goldschmidt; stearyl dimethicones, such as SilCare 41M65 sold by Clariant; C₂₀-C₂₄ alkyl dimethicones, such as SilCare 41M70 sold by Clariant; C₂₄-C₂₈ alkyl dimethicones, such as SilCare 41M80 sold by Clariant; and such as the silicone polymers disclosed in EP 0 545 002, the disclosure of which is hereby incorporated by reference.

In an embodiment, the at least one compatibilizing agent is present in the composition in an amount ranging from 5% to 90% by weight relative to the total weight of the composition. In another embodiment, the at least one compatibilizing agent is present in an amount ranging from 5% to 80% by weight relative to the total weight of the composition. In another embodiment, the at least one compatibilizing agent is present in an amount ranging from 5% to 60% by weight relative to the total weight of the composition.

Further, according to the present invention, the inventive composition, in certain embodiments, further comprises at least one low viscosity dimethicone different from the at least one dimethicone. Thus, in one embodiment, the at least one low viscosity dimethicone has lower viscosity than the at least one dimethicone of the inventive composition. Non-limiting examples of at least one low viscosity dimethicone include dimethicones having a viscosity lower than 100 cst. For example, the inventive emulsion may further comprise Dow Corning 1503 fluid which is a blend of an ultra high viscosity dimethiconol in a low viscosity dimethicone.

In an embodiment, the at least one low viscosity dimethicone is present in the composition in an amount ranging from 5% to 80% by weight relative to the total weight of the composition. In another embodiment, the at least one low viscosity dimethicone is present in an amount ranging from 10% to 60% by weight relative to the total weight of the composition.

Further, the inventive composition, in certain embodiments, further comprises at least one fluorosilicone. Non-limiting examples of at least one fluorosilicone include FL-05, FL-10, X-22-821, X-22-822, and FL100 (including FL100 (100 cst), FL100 (450 cst), FL100 (1000 cst), and FL100 (10,000 cst)), all of which are commercially available from Shin-Etsu.

In an embodiment, the at least one fluorosilicone is present in the composition in an amount ranging from 0.1% to 30% by weight relative to the total weight of the composition. In another embodiment, the at least one fluorosilicone is present in an amount ranging from 0.5% to 10% by weight relative to the total weight of the composition.

Further, the inventive composition may further comprise at least one wax different from the at least one linear hydrocarbon wax. Non-limiting examples of such waxes include waxes of natural origin, such as beeswax, modified beeswax, carnauba wax, candelilla wax, jojoba wax, ouricury wax, Japan wax, cork fiber wax, sugar cane wax, non-linear paraffin waxes, lignite wax, microcrystalline waxes, lanolin wax, montan wax and ozokerites, hydrogenated oils such as hydrogenated jojoba oil, jojoba esters, waxes obtained by Fischer-Tropsch synthesis, fatty acid esters, fatty acid glycerides, and silicone waxes different from the at least one compatibilizing agent.

In an embodiment, the at least one wax different from the at least one linear hydrocarbon wax is present in the composition in an amount ranging from 0.01% to 20% by weight relative to the total weight of the composition. In another embodiment, the at least one wax different from the at least one linear hydrocarbon wax is present in an amount ranging from 0.1% to 15% by weight relative to the total weight of the composition.

Further, the composition of the present invention may further comprise at least one coloring agent. The at least one coloring agent may be chosen from pigments, dyes, such as liposoluble dyes, nacreous pigments, and pearling agents. The at least one coloring agent may be chosen, for example, in order to obtain emulsions which give good coverage, that is, which do not allow a significant amount of the at least one keratin material to which it is applied to show through. The at least one coloring agent may also reduce any sticky feel of the emulsions.

Representative liposoluble dyes which may be used according to the present invention include Sudan Red, DC Red 17, DC Green 6, β-carotene, soybean oil, Sudan Brown, DC Yellow 11, DC Violet 2, DC Orange 5, annatto, and quinoline yellow. The liposoluble dyes, when present, generally have a concentration ranging up to 20% by weight of the total weight of the composition, such as from 0.0001% to 6%.

The nacreous pigments which may be used according to the present invention may be chosen from white nacreous pigments such as mica coated with titanium or with bismuth oxychloride, colored nacreous pigments such as titanium mica with iron oxides, titanium mica with ferric blue or chromium oxide, titanium mica with an organic pigment chosen from those mentioned above, and nacreous pigments based on bismuth oxychloride. The nacreous pigments, if present, may be present in the composition in a concentration ranging up to 50% by weight of the total weight of the composition, such as from 0.1% to 20%.

The pigments which may be used according to the present invention may be chosen from white, colored, inorganic, organic, polymeric, nonpolymeric, coated and uncoated pigments. Representative examples of mineral pigments include titanium dioxide, optionally surface-treated, zirconium oxide, zinc oxide, cerium oxide, iron oxides, chromium oxides, manganese violet, ultramarine blue, chromium hydrate, and ferric blue. Representative examples of organic pigments include carbon black, pigments of D & C type, and lakes based on cochineal carmine, barium, strontium, calcium, and aluminum. If present, the pigments may be present in the composition in a concentration ranging up to 40% by weight of the total weight of the composition, such as from 1% to 35%, -and further such as from 2% to 25%. In the case of certain products, the pigments, including nacreous pigments, may, for example, represent up to 50% by weight of the composition.

In an embodiment of the present invention, the inventive composition comprises at least one pigment. In another embodiment, the at least one pigment is chosen from encapsulated pigments. As used herein, "encapsulated pigments" refer to pigments which are encapsulated within at least one polymer. The capsule may release some of its contents, i.e., the enclosed pigment and dispersing solvent, if present, when pressure is applied to the emulsion comprising such encapsulated pigments. For example, when encapsulated pigments are comprised in a lip product emulsion, the capsule may release some of its contents when the lips are pressed together.

Accordingly, in an embodiment, the inventive compositions comprise at least one encapsulated pigment, wherein the encapsulated pigments are dispersed in at least one solvent. Thus, when pressure is applied to the emulsion, the capsule may release some of its contents and may thereby allow maintenance of at least one of color (from the pigments) and gloss (from the at least one solvent) on the lips. In an embodiment, the at least one solvent is chosen from emollients, such as, for example, silicone emollients, octyl dodecanol, and polyglyceryl-2 triisostearate. In another embodiment, the at least one solvent is chosen from non-volatile silicones. In another embodiment, the at least one solvent is phenyltrimethicone. Thus, the present invention also -provides, in an embodiment, compositions further comprising at least one encapsulated pigment wherein said at least one encapsulated pigment is dispersed in at least one silicone.

In an embodiment, the invention provides for a composition comprising at least one encapsulated pigment, wherein the at least one encapsulated pigment is dispersed in phenyltrimethicone. In another embodiment, the present invention provides a method for maintaining at least one property chosen from color and gloss of a composition on at least one keratinous material, comprising applying a composition comprising at least one encapsulated pigment, wherein the at least one encapsulated pigment is dispersed in at least one silicone.

The maintenance of gloss of a composition over time may be measured by visual inspection after a specific amount of time. For example, gloss is maintained if the gloss of the composition on the keratinous material 30 minutes after application of the composition to the keratinous material is the, equal to or greater than the gloss of the same composition upon application as viewed by the naked eye.

The maintenance of color of a composition over time may be measured by visual inspection after a specific amount of time. For example, color is maintained if the color of the composition on the keratinous material 30 minutes after application of the composition to the keratinous material is as intense or more intense than the color of the same composition upon application as viewed by the naked eye. Further, L values of the composition can be measured (for example, using Minolta Chroma Meter CR-300) to determine the intensity of the color. In the cosmetic arts, and as defined in the L, a, b colorimetric notations system of the Commission Internationale de l'Eclairage, L defines the intensity of the shade. See U.S. Patent No. 6,010,541, Col 1, line 66 to Col. 2, line 8, and Col. 9, lines 15 - 57. The shade is proportionally more intense the lower the value of L (0 = black, 100 = white).

Accordingly to the present invention, the inventive compositions may further comprise at least one filler. As used herein, the term "filler" means any particle that is solid at room temperature and atmospheric pressure, used alone or in combination, which does not react chemically with the various ingredients of the emulsion and which is insoluble in these ingredients, even when these ingredients are heated to a temperature above room temperature and, in particular, to their softening point or their melting point. In an embodiment, the at least one filler has a melting point at least greater than 170°C, for example, greater than 200°C. In an embodiment, the at least one filler may have an apparent diameter ranging from 0.01 µm to 150 µm, such as from 0.5 µm to 120 µm, for example from 1 µm to 80 µm. An apparent diameter corresponds to the diameter of the circle into which the elementary particle fits along its shortest dimension (thickness for leaflets). Further, the at least one filler may be absorbent, *i.e*., capable in particular of absorbing the oils of the composition and also the biological substances secreted by the skin, may be surface-treated, *e.g*., to make it lipophilic, and/or may be porous so as to absorb -the sweat and/or sebum secreted by the skin.

The at least one filler may be chosen from inorganic and organic fillers, and may have any shape such as lamellar, spherical and/or oblong. Non-limiting examples of the at least one inert filler include talc, mica, silica, kaolin, polyamide powders (such as Nylon® powder, and such as the product sold by Atochem as Orgasol®), poly-β-alanine powders, polyethylene powders, acrylic polymer powders (such as polymethyl methacrylate (PMMA) powder, for instance the product sold by Wacker as Covabead LH-85 (particle size 10-12 µm) and the acrylic acid copolymer powder sold by Dow Corning as Polytrap®), polytetrafluoroethylene (Teflon®) powders, lauroyllysine, boron nitride, silica, kaolin, starch, starch derivatives, hollow polymer microspheres (such as those hollow polymer microspheres formed from polyvinylidene chloride and acrylonitrile, for instance the product sold by Nobel Industrie as Expancel®), and polymerized silicone microspheres (such as those polymerized silicone microspheres sold by Toshiba as Tospearl®), precipitated calcium carbonate, magnesium carbonate and hydrocarbonate, hydroxyapatite, hollow silica microspheres (such as the product sold by Maprecos as Silica Beads®), glass microcapsules, ceramic microcapsules, and polyester particles.

Depending on the intended application, such as a stick, hardness of the composition may also be considered. In an embodiment, the at least one compatibilizing agent is present in an amount effective to further provide hardness to the composition. The hardness of a composition may, for example, -be expressed in grams (g). The composition of the present invention may, for example, have a hardness ranging from 10 g to 5000 g, such as from 15 g to 500 g, further such as from 20 g to 600 g, and further such as from 30 g to 150 g.

This hardness is measured as follows. A test for hardness is according to a method of penetrating a probe into the composition and in particular using a texture analyzer (for example TA-XT2i from Stable Microsystems) equipped with a stainless steel cylinder of height 35 mm and diameter 4 mm. The hardness measurement is carried out at 20°C at the center of 5 samples of the composition. The cylinder penetrates at a speed of 2 mm/s, the total displacement being 5 mm. The recorded hardness value is that of the maximum peak observed. The measurement error is ± 10g.

The hardness of the composition of the present invention may be such that the compositions are self-supporting and can easily disintegrate to form a satisfactory deposit on a keratinous material. In addition, this hardness may impart good impact strength to the inventive composition which may be molded or cast, for example, in stick or dish form.

The skilled artisan may choose to evaluate a composition using the tests for hardness outlined above based on the application envisaged and the hardness desired.

According to the present invention, the composition in stick form may also possess the properties of deformable, flexible elastic solids and may also have noteworthy elastic softness upon application to a keratinous material. Further, in an embodiment, the inventive composition has a melting point ranging from 40°C to 150°C.

The composition of the present invention may also further comprise at least one suitable additive commonly used in the field concerned. In an embodiment, the at least one additive may be chosen from fatty materials, coloring agents, humectants, texture modifiers, antifoaming agents, moisturizers, viscosity modifiers, antioxidants, essential oils, preserving agents, fragrances, neutralizing agents, liposoluble polymers, polysaccharides, fluorinated compounds, and cosmetically active agents and dermatological active agents such as, for example, emollients, vitamins, plant extracts, essential fatty acids, and UV-screening agents. In an embodiment, the composition of the present invention is transparent and/or clear, including, for example, a composition without pigments. In yet another embodiment, the composition of the present invention is neither transparent nor clear.

Needless to say, the person skilled in the art will take care to select the optional additional additives and the amount thereof such that at least one advantageous property of the composition according to the invention, such as stability or non-migration, is not, or is not substantially, adversely affected by the addition(s) envisaged.

Other than in the operating examples, or where otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and in the attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. The following examples are intended to illustrate the invention without limiting the scope as a result. The percentages are given on a weight basis.

### Examples

### Example

The following compositions were prepared.

**[0094] Table 1.**

| **Components** | **Inventive Composition 1 (% wt)** | **Inventive Composition 2 (% wt)** | **Inventive Composition 3 (% wt)** |
|---|---|---|---|
| ***Phase A*** | | | |
| Dimethicone¹ | 30.0 | 30.0 | 30.0 |
| Dimethiconol and dimethicone² | 58.0 | 56.0 | 56.0 |
| Trifluoropropyl dimethicone³ | -- | 1.0 | 1.0 |
| | | | |

| ***Phase B*** | | | |
|---|---|---|---|
| C₃₀-C₄₅ alkyl dimethicone⁴ | 7.0 | 7.0 | 5.0 |
| Polyethylene wax⁵ | 5.0 | 6.0 | 8.0 |

| | | | |
|---|---|---|---|
| ¹ Dow Corning 200 (350 cst) | | | |
| ² Dow Corning 1503 Fluid | | | |
| ³ FL-5 | | | |
| ⁴ SF1642 | | | |
| ⁵ Polyethylene 500 | | | |

### Preparation

The components of phase A were mixed until uniform and then heated in an oil bath. When the temperature of the oil bath reached 110°C, C₃₀-C₄₅ alkyl dimethicone was added to the phase A mixture and the resulting mixture was stirred until uniform. The polyethylene wax was then slowly added to the resulting mixture while maintaining the temperature of the oil bath at 110°C until the wax was completely melted and dispersed. The temperature of the mixture was lowered to 90°C-95°C at which time the bulk was poured into a mold.

Inventive Composition 1 was harder than Inventive Composition 2 and Inventive Composition 3 was harder than Inventive Composition 2.

### Results

### As Lip Product

Lip compositions were applied to the bare lips of eight sensory panelists trained in the application and evaluation of lip products and each was evaluated for various properties at 4 and 6 hours. The panelists followed the following protocol:

Panelists used standard soap to wash their lips before product application and allowed their lips to equilibrate for 10 minutes. If there was a base coat, panelists applied base coat to lips as follows: starting at the upper lip, center: two strokes to the left, then two strokes to the right; starting at the left corner of lower lip: two strokes from left to right then right to left. Panelists answered a questionnaire immediately after application. At 1 minute, the panelists checked for tack and grease. At 2 minutes, the panelists tested for product transfer by kissing a white piece of paper. At 3 minutes, the panelists checked for tack and grease. At 5 minutes, the panelists tested for product transfer by kissing a white piece of paper. The panelists would then apply the topcoat as follows: starting at the upper lip, center: two strokes to the left, then two strokes to the right; starting at the left corner of lower lip: two strokes from left to right then right to left. Panelists answered a questionnaire at 4 hours and at 6 hours. Tukey's Studentized Range test statistics were used for analysis.

The results are presented in Table 2.

**Table 2.**

| **Results** | **Inventive Composition 1** | **Inventive Composition 2** | **Inventive Composition 3** | **Lipfinity TopCoat*** |
|---|---|---|---|---|
| Ease of application | 7.7 | 7.7 | 7.9 | 7.5 |
| Richness of feel | 7.5 | 6.6 | 7.5 | 6.8 |
| Slip on lips | 7.7 | 7.6 | 8.0 | 7.4 |

| | | | | |
|---|---|---|---|---|
| *Commercially available from Max Factor | | | | |

The results show that each of the inventive compositions, Inventive Composition 1, Inventive Composition 2, and Inventive Composition 3, were easier to apply, and felt richer and silkier on lips than Lipfinity TopCoat.

As Topcoat over Basecoat in Stick Form

The following basecoat composition in stick form, Stick Basecoat, was prepared.

**Table 3.**

| **Components** | **Stick Basecoat** |
|---|---|
| Isododecane | q.s. |
| Film Formers | 20 |
| Wax | 11 |
| Emollients | 5.3 |
| Plasticizer | 2.3 |
| Pigments and fillers | 7.9 |

### Results

The basecoat composition above, Stick Basecoat, was applied to the bare lips of eight sensory panelists trained in the application and evaluation of lip products and each was evaluated for various properties, as described above. A film of one of the inventive compositions, Inventive Composition 1 or Inventive Composition 2, was then applied to the basecoated lips as a topcoat. Various properties of the double coated lips were evaluated. Both of the inventive compositions, Inventive Composition 1 and Inventive Composition 2, increased the stickiness on lips, provided a richer, creamier, thicker feel gloss on the lips, and reduced the tight, dry feeling on lips as compared to the lips having only a basecoat composition. The wear comfort of the basecoat was increased by applying either Inventive Composition 1 or Inventive Composition 2 of the present invention over the basecoat. The gloss/shine and moisture also increased with application of the invention composition.

### As Topcoat over Liquid Basecoat

The following liquid basecoat composition, Liquid Basecoat, was prepared.

**Table 4.**

| **Components** | **Liquid Basecoat** |
|---|---|
| Isododecane | q.s. |
| Emollients | 4.3 |
| Plasticizer | 20 |
| Film Formers | 13.6 |
| Pigments & Fillers | 2.75 |

### Results

The basecoat composition above, Liquid Basecoat, was applied to the bare lips of eight sensory panelists trained in the application and evaluation of lip products and each was evaluated for various properties. A film of Inventive Composition 3 was then applied to the basecoated lips as a topcoat. Various properties of the double coated lips were evaluated. Inventive Composition 3, 1 or 2 was found to increase the stickiness on lips, provide a richer, creamier, thicker feel on the lips, and reduce the tight, dry feeling on lips as compared to the lips having only a basecoat composition. The wear comfort of the basecoat was increased by applying Inventive Composition 3, 1 or 2 of the present invention over the basecoat. The gloss/shine increased and the dry effect decreased upon application of the inventive composition, resulting in improved qualities.

Thus, the above results show that the inventive compositions, Inventive Composition 1, Inventive Composition 2, and Inventive Composition 3, provide at least one desirable property to lips coated with a basecoat composition, wherein the basecoat composition is in the form of a stick or a liquid.

### Comparative Examples

The following comparative compositions were prepared.

**Table 5.**

| **Components** | **Comparative Composition 4 (% wt)** | **Comparative Composition 5 (% wt)** | **Comparative Composition 6 (% wt)** |
|---|---|---|---|
| ***Phase A*** | | | |
| Dimethicone (20 cst) | 44.0 | 44.0 | 30.0 |
| Dimethicone (500 cst) | -- | -- | 29.0 |
| Phenyl trimethicone (1000 cst) | 44.0 | 44.0 | -- |
| Cetyl dimethicone (1000 cst) | -- | | 28.0 |
| | | | |

| ***Phase B*** | | | |
|---|---|---|---|
| C₃₀-C₄₅ alkyl dimethicone¹ | 8.0 | 6.0 | 7.0 |
| C₃₀-C₄₅ alkyl methicone² | -- | 6.0 | |
| Ozokerite wax | 4.0 | -- | -- |
| Candelilla hydrocarbon wax³ | -- | | 6.0 |

| | | | |
|---|---|---|---|
| ¹ SF1642 | | | |
| ² AMS-C30 wax | | | |
| ³ Candelilla FC-31 (hydrocarbon fraction of candelilla wax from Japan Natural Products) | | | |

### Preparation

The components of phase A were mixed until uniform and then heated in an oil bath. When the temperature of the oil bath reached 110°C, C₃₀-C₄₅ alkyl dimethicone and/or C₃₀-C₄₅ alkyl methicone, if present, was/were added to the phase A mixture and the resulting mixture was stirred until uniform. The wax, if present, was then slowly added to the resulting mixture while maintaining the temperature of the oil bath at 110°C until the wax was completely melted and dispersed. The temperature of the mixture was lowered to 90°C-95°C at which time the bulk was poured into a mold, if possible.

### Results

Comparative Composition 4: This composition, although capable of being molded into a stick, resulted in the non-linear wax (ozokerite) becoming grainy in the stick thereby indicating instability of the composition and the absence of an emulsion. The film deposited on skin when this stick was applied to skin was rough and did not have a powdery feel.

Comparative Composition 5: This composition was incapable of being molded into a stick as it was too soft. This demonstrates that at least one dimethicone and at least one compatibilizer were insufficient to obtain the desired properties without at least one linear hydrocarbon wax.

Comparative Composition 6: This composition was incapable of being molded into a stick as it was too soft. While candelilla FC-31 is a hydrocarbon wax, it includes esters, acids and some branched wax.

## Claims

1. A composition comprising an emulsion comprising at least one dimethicone, at least one linear hydrocarbon wax, and at least one compatibilizing agent.

2. A composition according to claim 1, wherein said composition is capable of being molded in the form of a stick.

3. A composition according to claim 2, wherein said stick is stable.

4. A composition according to claim 1, wherein said emulsion is chosen from aqueous emulsions and non-aqueous emulsions.

5. A composition according to claim 1, wherein said at least one dimethicone is chosen from dimethicones of viscosity equal to or higher than 100 cst.

6. A composition according to claim 5, wherein said at least one dimethicone is chosen from dimethicones of viscosity equal to or higher than 350 cst.

7. A composition according to claim 6, wherein said at least one dimethicone is chosen from dimethicones of viscosity equal to or higher than 500 cst.

8. A composition according to claim 1, wherein said at least one dimethicone is present in said composition in an amount ranging from 5% to 95% by weight relative to the total weight of said composition.

9. A composition according to claim 8, wherein said at least one dimethicone is present in said composition in an amount ranging from 20% to 80% by weight relative to the total weight of said composition.

10. A composition according to claim 1, wherein said at least one linear hydrocarbon wax is solid at room temperature.

11. A composition according to claim 10, wherein said at least one linear hydrocarbon wax has a melting point greater than 35°C.

12. A composition according to claim 11, wherein said at least one linear hydrocarbon wax has a melting point greater than 55°C.

13. A composition according to claim 1, wherein said at least one linear hydrocarbon wax is incompatible with at least one dimethicone of viscosity equal to or greater than 100 cst.

14. A composition according to claim 13, wherein said at least one linear hydrocarbon wax is incompatible with at least one dimethicone of viscosity equal to or greater than 350 cst.

15. A composition according to claim 14, wherein said at least one linear hydrocarbon wax is incompatible with at least one dimethicone of viscosity equal to or greater than 500 cst.

16. A composition according to claim 1, wherein said at least one linear hydrocarbon wax is chosen from polyethylene waxes, linear paraffin waxes, and long-chain linear alcohols.

17. A composition according to claim 1, wherein said at least one linear hydrocarbon wax is chosen from substituted linear alkanes, unsubstituted linear alkanes, substituted linear alkenes, and unsubstituted linear alkenes.

18. A composition according to claim 1, wherein said at least one linear hydrocarbon wax is present in said composition in an amount ranging from 2% to 30% by weight relative to the total weight of said composition.

19. A composition according to claim 18, wherein said at least one linear hydrocarbon wax is present in said composition in an amount ranging from 5% to 20% by weight relative to the total weight of said composition.

20. A composition according to claim 1, wherein said at least one compatibilizing agent is chosen from silicone polymers comprising at least one hydrocarbon group.

21. A composition according to claim 20, wherein said silicone polymers comprising at least one hydrocarbon group are chosen from linear polysiloxanes comprising at least one hydrocarbon group, branched polysiloxanes comprising at least one hydrocarbon group, and cyclic linear polysiloxanes comprising at least one hydrocarbon group.

22. A composition according to claim 20, wherein said at least one of said at least one hydrocarbon groups comprises at least 18 carbon atoms.

23. A composition according to claim 1, wherein said at least one compatibilizing agent is chosen from compatibilizing agents of formula (I): 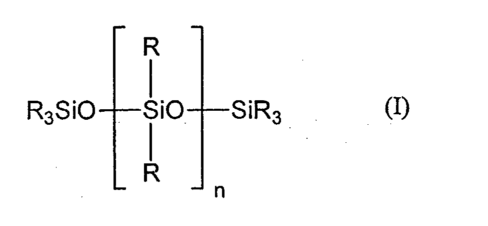 wherein
R, which may be identical or different, are each chosen from hydrocarbon groups;
the total molecular weight of Si and O atoms is at least 10% of the total weight of said at least one compatibilizing agent of formula (I), and
the melting point of said at least one compatibilizing agent of formula (I) ranges from 35°C to 110°C
with the proviso that at least one R comprises at least 18 carbon atoms..

24. A composition according to claim 23, wherein said at least one R is chosen from C₃₀ to C₄₅ hydrocarbon groups.

25. A composition according to claim 23, wherein said at least one R is chosen from C₃₀ to C₄₅ alkyl groups.

26. A composition according to claim 1, wherein said at least one compatibilizing agent is chosen from linear polysiloxanes comprising at least one C₃₀-C₄₅ alkyl group, C₃₀-C₄₅ alkyl methicones, C₂₀-C₂₄ alkyl methicones, C₂₄-C₂₈ alkyl methicones, hexyl methicones, caprylyl methicones, lauryl methicones, stearyl methicones, cetyl dimethicone, stearyl dimethicones, C₂₀-C₂₄ alkyl dimethicones, and C₂₄-C₂₈ alkyl dimethicones.

27. A composition according to claim 1, wherein said at least one compatibilizing agent is chosen from at least one C₃₀-C₄₅ alkyl dimethicone.

28. A composition according to claim 1, wherein said at least one compatibilizing agent is present in said composition in an amount ranging from 5% to 90% by weight relative to the total weight of said composition.

29. A composition according to claim 28, wherein said at least one compatibilizing agent is present in said composition in an amount ranging from 5% to 60% by weight relative to the total weight of said composition.

30. A composition according to claim 1, wherein said composition further comprises at least one low viscosity dimethicone different from said at least one dimethicone.

31. A composition according to claim 30, wherein said at least one low viscosity dimethicone has a lower viscosity than said at least one dimethicone.

32. A composition according to claim 31, wherein said at least one low viscosity dimethicone is chosen from dimethicones of viscosity lower than 100 cst.

33. A composition according to claim 32, wherein said at least one low viscosity dimethicone is present in said composition in an amount ranging from 5% to 80% by weight relative to the total weight of said composition.

34. A composition according to claim 1, wherein said at least one low viscosity dimethicone is present in said composition in an amount ranging from 10% to 60% by weight relative to the total weight of said composition.

35. A composition according to claim 1, wherein said composition further comprises at least one fluorosilicone.

36. A composition according to claim 1, wherein said composition further comprises at least one wax different from said at least one linear hydrocarbon wax.

37. A composition according to claim 1, wherein said composition further comprises at least one coloring agent.

38. A composition according to claim 37, wherein said at least one coloring agent is chosen from encapsulated pigments.

39. A composition according to claim 38, wherein said encapsulated pigments are dispersed in at least one solvent.

40. A composition according to claim 39, wherein said at least one solvent is phenyltrimethicone.

41. A composition according to claim 1, wherein said composition further comprises at least one filler.

42. A composition according to claim 1, wherein said composition further comprises at least one additive chosen from fatty materials, coloring agents, humectants, texture modifiers, antifoaming agents, moisturizers, viscosity modifiers, antioxidants, essential oils, preserving agents, fragrances, neutralizing agents, liposoluble polymers, polysaccharides, fluorinated compounds, cosmetically active agents, and dermatological active agents.

43. A composition according to claim 1, wherein said composition has a melting point ranging from 40°C to 150°C.

44. A mascara product, an eyeliner product, a foundation product, a lipstick product, a blush for cheeks or eyelids, a deodorant product, a make-up product for the body and/or hair, a make-up-removing product, an eyeshadow product, a face powder product, a concealer product, a treating shampoo product, a hair conditioning product, a sunscreen, colorant for the skin or hair, or skin care formula comprising an emulsion comprising at least one dimethicone, at least one linear hydrocarbon wax, and at least one compatibilizing agent.

45. A care, make-up, and/or treatment composition for the skin and/or the lips of the face and/or for superficial body growths comprising an emulsion comprising at least one dimethicone, at least one linear hydrocarbon wax, and at least one compatibilizing agent.

46. A non-aqueous stick composition comprising an emulsion comprising at least one dimethicone of viscosity equal to or greater than 350 cst, at least one linear polyethylene wax, and at least one C₃₀-C₄₅ alkyl dimethicone.

47. A method for modifying the structure of a composition comprising at least one dimethicone and at least one linear hydrocarbon wax, comprising including at least one compatibilizing agent in an amount effective to mold said composition into a stick.

48. A method of increasing at least one property of a basecoat composition chosen from properties of staying power, long wear properties, glossiness, and wear comfort comprising
applying to at least one keratinous material at least one basecoat composition; and
applying to said basecoated keratinous material a composition comprising an emulsion comprising at least one dimethicone, at least one linear hydrocarbon wax, and at least one compatibilizing agent.

49. A method for maintaining at least one property chosen from color and gloss of a composition on at least one keratinous material, comprising applying a composition comprising at least one encapsulated pigment, wherein the at least one encapsulated pigment is dispersed in at least one silicone.
